(19) Europäisches Patentamt
European Patent Office
Office européen des brevets

(11) Veröffentlichungsnummer: **0 308 781**
**A2**

(12) # EUROPÄISCHE PATENTANMELDUNG

(21) Anmeldenummer: **88114932.2**

(22) Anmeldetag: **13.09.88**

(51) Int. Cl.[4]: **A61K 31/41 , A61K 31/415 , C07D 249/08 , C07D 233/60**

(30) Priorität: **25.09.87 DE 3732387**

(43) Veröffentlichungstag der Anmeldung: **29.03.89 Patentblatt 89/13**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Stroech, Klaus, Dr.**
**Rolsberger Strasse 22**
**D-5650 Solingen 19(DE)**
Erfinder: **Plempel, Manfred, Dr.**
**Zwengenberger Strasse 3c**
**D-5657 Haan(DE)**

(54) **Antimykotische Mittel.**

(57) Die vorliegende Erfindung betrifft neue Azolylmethylcyclopropyl-Derivate zur Behandlung von Krankheiten, insbesondere Mykosen.

EP 0 308 781 A2

## Antimykotische Mittel

Die vorliegende Erfindung betrifft neue Azolylmethylcyclopropyl-Derivate sowie deren pharmakologisch verträgliche Säureadditions-Salze zur Behandlung von Krankeiten, insbesondere Mykosen.

Es ist bereits allgemein bekannt geworden, daß bestimmte Azolylmethyl-cyclopropyl-carbinol-Derivate, wie beispielsweise 1-(4-Chlorphenyl)-1-(1-fluor-cycloprop-1-yl)-2-(1,2,4-triazol-1-yl)-ethan-1-ol und 1-(1-Fluorcycloprop-1-yl)-1-phenyl-2-(1,2,4-triazol-1-yl)-ethan-1-ol, antimykotische Eigenschaften aufweisen (vergleiche EP-OS 0 180 850). Die Wirkung dieser Stoffe ist jedoch nicht immer in allen Indikationsbereichen voll zufriedenstellend.

Es wurde gefunden, daß die neuen Azolylmethyl-cyclopropyl-Derivate der Formel

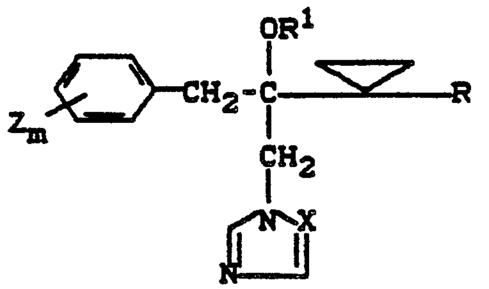

(I)

in welcher

R für Halogen, Alkyl, gegebenenfalls substituiertes Phenyl oder für die Gruppierung $-Y-R^2$ steht, worin

Y für Sauerstoff, Schwefel, SO oder $SO_2$ steht und

$R^2$ für gegebenenfalls substituiertes Phenyl steht,

$R^1$ für Wasserstoff, Alkyl oder Acyl steht,

X für Stickstoff oder eine CH-Gruppe steht,

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und

m für die Zahlen 0, 1, 2 oder 3 steht,

sowie deren Säureadditions-Salze gute antimikrobielle, insbesondere antimykotische Eigenschaften aufweisen.

Die erfindungsgemäß zu verwendenden Azolylmethyl-cyclopropyl-Derivate sind durch die Formel (I) allgemein definiert. In dieser Formel stehen vorzugsweise

R für Fluor, Chlor, Brom, Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Y-R^2$, worin

Y für Sauerstoff, Schwefel, SO oder $SO_2$ steht und

$R^2$ für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht,

$R^1$ für Wasserstoff, Alkyl mit 1 bis 6 Kohlenstoffatomen oder Alkylcarbonyl mit 1 bis 6 Kohlenstoffatomen in der Alkylgruppe,

X für Stickstoff oder eine CH-Gruppe,

Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy und

m für die Zahlen 0, 1, 2 oder 3.

Wenn m für die Zahlen 2 oder 3 steht, können die für Z stehenden Reste gleich oder verschieden sein.

Besonders bevorzugt sind diejenigen Verbindungen der Formel (I), in denen

R für Fluor, Chor, Brom, Methyl, Ethyl, gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder für die Gruppierung -Y-$R^2$ steht, worin

Y für Sauerstoff, Schwefel, SO oder $SO_2$ steht und

$R^2$ für gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht,

$R^1$ für Wasserstoff, Methyl, Ethyl, n-Propyl, Isopropyl, n-Butyl, Isobutyl, Methylcarbonyl, Ethylcarbonyl, n-Propylcarbonyl, Isopropyl-carbonyl, n-Butylcarbonyl und Isobutyl-carbonyl steht,

X für Stickstoff oder eine CH-Gruppe steht,

Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy steht und

m für die Zahlen 0, 1, 2 oder 3 steht.

Bevorzugte erfindungsgemäße Verbindungen sind auch pharmakologisch verträgliche Additionsprodukte aus Säuren und denjenigen Azolylmethyl-cyclopropyl-Derivaten der Formel (I), in denen R, $R^1$, X, Z und m die Bedeutungen haben, die bereits vorzugsweise für diese Reste bzw. diesen Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Als Beispiele für Azolylmethyl-cyclopropyl-Derivate der Formel (I) seien die in der folgenden Tabelle aufgeführten Stoffe gennant.

**Tabelle**

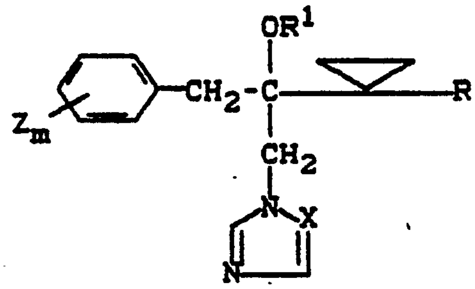

(I)

| $Z_m$ | $R^1$ | X | R | Schmelzpunkt (° C) |
|---|---|---|---|---|
| 4-Cl | H | N | Cl | 97 |
| 2,4-$Cl_2$ | " | " | Cl | 159 |
| 2,4-$F_2$ | " | " | Cl | 117 |
| 4-$CH_3$ | " | " | Cl | 132 |
| 4-$CF_3$ | " | " | Cl | |
| 4-$OCF_3$ | " | " | Cl | |
| 4-$OCH_3$ | " | " | Cl | |
| 4-$SCH_3$ | " | " | Cl | |
| 2,4,6-$Cl_3$ | " | " | Cl | |
| 4-Cl | " | " | F | |
| 4-Cl | " | CH | Cl | |
| 4-Cl | $CH_3$ | N | Cl | |
| 4-Cl | H | " | 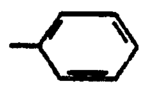 | |
| 4-Cl | " | " | 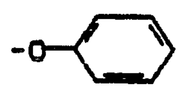 | |
| 4-Cl | " | " | 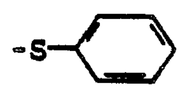 | |
| 2-F | " | " | Cl | 103 |
| 2-Cl | " | " | " | 108 |
| 3,4-$Cl_2$ | " | " | " | 80-92 |
| 3,4-$F_2$ | " | " | " | 98 |
| 4-F | " | CH | " | 171 |

**Tabelle** (Fortsetzung)

| $Z_m$ | $R^1$ | X | R | (° C) Schmelzpunkt |
|---|---|---|---|---|
| 4-Cl | " | " | 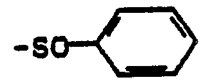 | |
| 4-Cl | " | " | 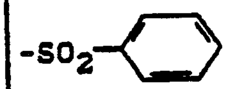 | |
| 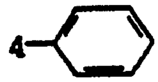 | " | " | Cl | |
| 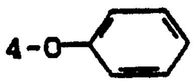 | " | " | Cl | |
| $4-C_4H_9-t.$ | " | " | Cl | |
| $2-Cl, 4-CH_3$ | " | " | Cl | |
| - | " | " | Cl | 81 |
| 4-Cl | $-CO-CH_3$ | " | Cl | |
| 4-Cl | $-C_2H_5$ | " | Cl | |
| 4-F | $CH_3$ | " | F | |
| 4-Cl | H | " | $CH_3$ | |

Die erfindungsgemäß zu verwendenden Azolylmethylcyclopropyl-Derivate der Formel (I) sowie ihre Säureadditions-Salze sind noch nicht bekannt. Sie sind jedoch Gegenstand einer noch nicht veröffentlichten Deutschen Patentanmeldung (P 37 20 755). Sie können nach den dort beschriebenen Verfahren erhalten werden, indem man

a) Propanol-Derivate der Formel

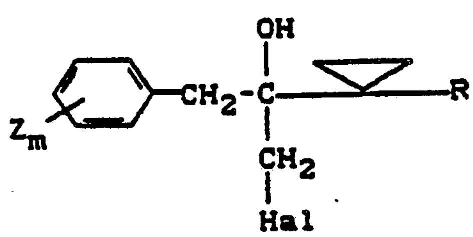

(II)

R, Z und m die oben angegebene Bedeutung haben und Hal für Chlor, Brom oder Jod steht, mit Azolen der Formel

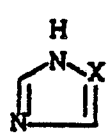

(III)

in welcher

X die oben angegebene Bedeutung hat,
in Gegenwart eines Säurebindemittels und in Gegenwart eines Verdünnungsmittels umsetzt; oder

b) Oxirane der Formel

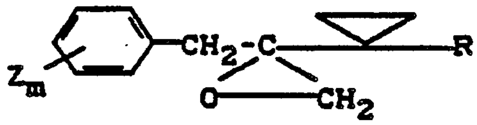

(IV)

in welcher
R, Z und m die oben angegebene Bedeutung haben,
mit Azolen der Formel

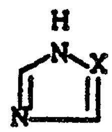

(III)

in welcher
X die oben angegebene Bedeutung hat,
in Gegenwart eines Säurebindemittels und in Gegenwart eines Verdünnungsmittels umsetzt, oder

c) Azolylmethyl-ketone der Formel

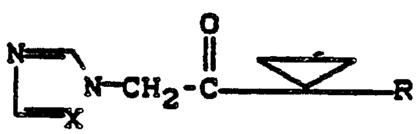

(V)

in welcher
R und X die oben angegebene Bedeutung haben,
mit metallorganischen Verbindungen der Formel

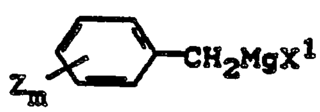

(VI)

in welcher
Z und m die oben angegebene Bedeutung haben und
$X^1$ für Chlor, Brom oder Iod steht,
in Gegenwart eines Verdünnungsmittels umsetzt, oder

d) Azolylmethyl-cyclopropyl-carbinol-Derivate der Formel

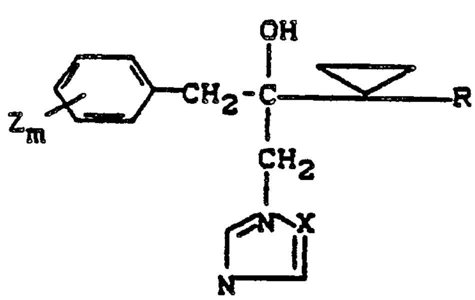

(Ia)

in welcher
R, X, Z und m die oben angegebene Bedeutung haben,
mit starken Basen in Gegenwart eines Verdünnungsmittels umsetzt und die dabei entstehenden Alkoholate der Formel

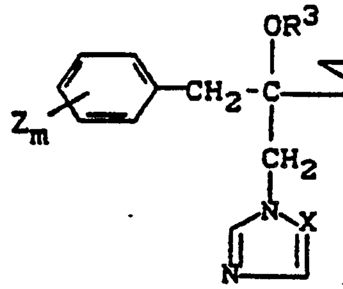

(Ib)

in welcher
R, X, Z und m die oben angegebene Bedeutung haben und
$R^3$ für einen kationischen Rest einer Base steht,
mit Halogenverbindungen der Formel

$R^4$-Hal′ (VII)

in welcher
$R^4$ für Alkyl oder Acyl steht und
Hal′ für Halogen steht,
in Gegenwart eines Verdünnungsmittels umsetzt,
und gegebenenfalls anschließend an die so erhaltenen Verbindungen der Formel (I) in üblicher Weise eine Säure oder ein Metallsalz addiert.

Die erfindungsgemäßen Stoffe enthalten ein asymmetrisch substituiertes Kohlenstoffatom. Sie können daher in optischen Isomerenformen anfallen. Die vorliegende Erfindung betrifft sowohl die einzelnen Isomeren als auch deren Gemisch.e

Verwendet man 1-Chlor-2-(1-chlorcyclopropyl)-3-(4-fluorphenyl)-propan-2-ol und 1,2,4-Triazol als Ausgangsstoffe, so kann der Verlauf des erfindungsgemäßen Verfahrens (a) durch das folgende Formelschema veranschaulicht werden:

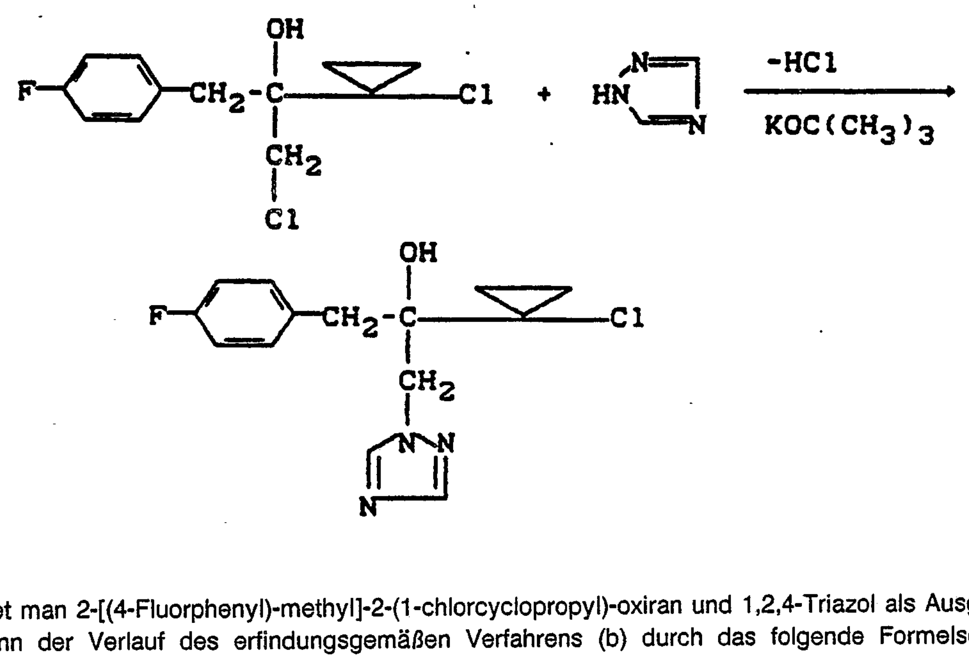


Verwendet man 2-[(4-Fluorphenyl)-methyl]-2-(1-chlorcyclopropyl)-oxiran und 1,2,4-Triazol als Ausgangsstoffe, so kann der Verlauf des erfindungsgemäßen Verfahrens (b) durch das folgende Formelschema veranschaulicht werden:

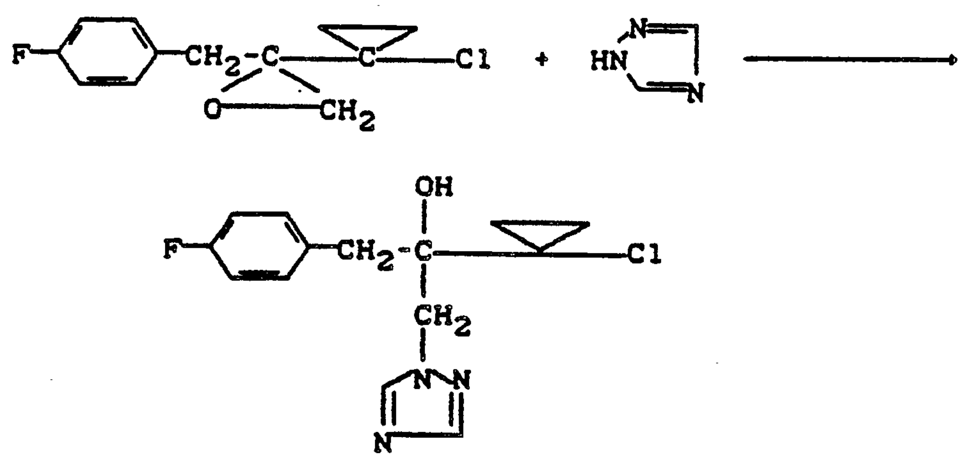

Verwendet man (1,2,4-Triazol-1-yl-methyl)-(1-chlorcycloprop-1-yl)-keton und 4-Fluorbenzyl-magnesiumbromid als Ausgangsstoffe, so kann der Verlauf des erfindungsgemäßen Verfahrens (c) durch das folgende Formelschema veranschaulicht werden:

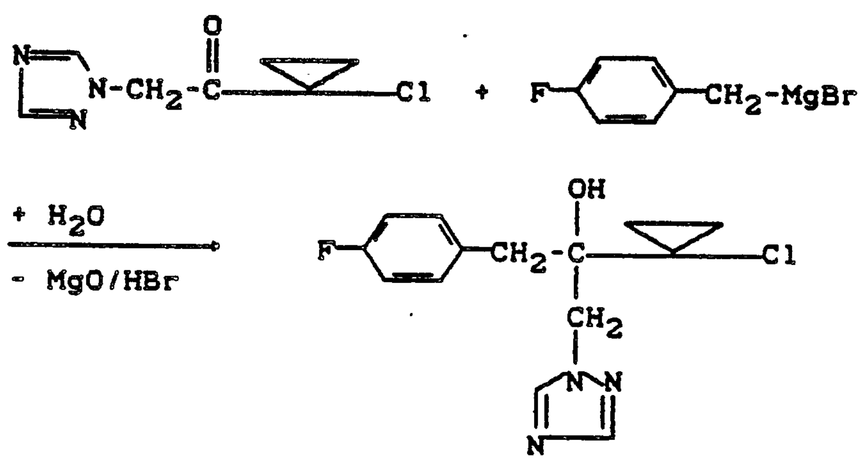


Verwendet man 1-(4-Fluorphenyl)-2-(1-chlorcyclopropyl)-3-(1,2,4-triazol-1-yl)-propan-2-ol und Natriumhydrid als Ausgangsstoffe und Iodmethan als Reaktionskomponente, so kann der Verlauf des erfindungsgemäßen Verfahrens (d) durch das folgende Formelschema veranschaulicht werden:

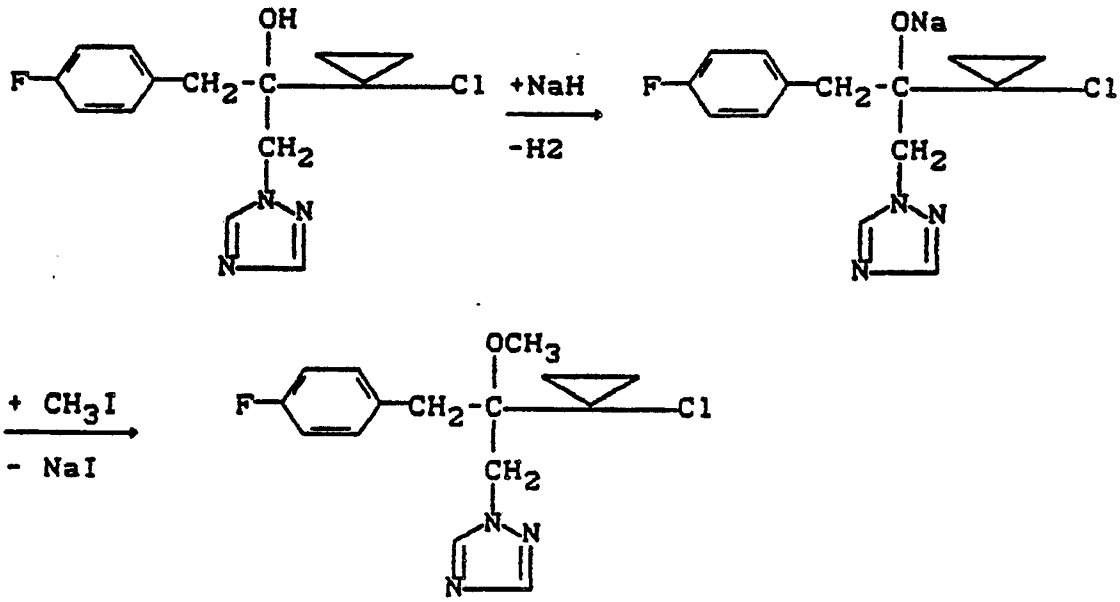


Die bei dem erfindungsgemäßen Verfahren (a) als Ausgangsstoffe benötigten Propanol-Derivate sind durch die Formel (II) allgemein definiert. In dieser Formel haben R, Z und m vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden. Hal steht vorzugsweise für Chlor oder Brom.

Die Propanol-Derivate der Formel (II) sind bisher noch nicht bekannt. Sie lassen sich herstellen, indem man Cyclopropylketone der Formel

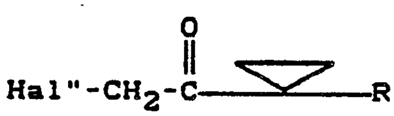
(VIII)

in welcher
R die oben angegebene Bedeutung hat und
Hal″ für Chlor oder Brom steht,
mit metallorganischen Verbindungen der Formel

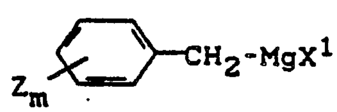
(VI)

in welcher
$X^1$, Z und m die oben angegebene Bedeutung haben und
in Gegenwart eines Verdünnungsmittels umsetzt.

Die bei der Herstellung der Propanol-Derivate nach dem obigen Verfahren als Ausgangsstoffe benötigten Cyclopropylketone der Formel (VIII) sind teilweise bekannt. Sie lassen sich herstellen, indem man Ketone der Formel

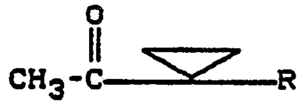
(IX)

in welcher

R die oben angegebene Bedeutung hat,
mit Chlorierungsmitteln oder Bromierungsmitteln in Gegenwart eines Verdünnungsmittels umsetzt.

Die bei der Herstellung von Cyclopropylketonen der Formel (VIII) als Ausgangsstoffe benötigten Ketone der Formel (IX) sind bekannt oder lassen sich nach prinzipiell bekannten Verfahren synthetisieren (vgl. Synthesis 1977, 189).

Als Chlorierungsmittel und Bromierungsmittel kommen bei dem obigen Verfahren zur Herstellung von Cyclopropylketonen der Formel (VIII) alle für derartige Umsetzungen üblichen Chlorierungs- und Bromierungs-Reagenzien in Betracht. Vorzugsweise verwendbar sind Sulfurylchlorid, Sulfurylbromid und Brom.

Als Verdünnungsmittel kommen bei der Herstellung von Cyclopropylketonen der Formel (VIII) nach dem obigen Verfahren alle für derartige Umsetzungen üblichen inerten organischen Solventien infrage. Vorzugsweise verwendbar sind halogenierte aliphatische Kohlenwasserstoffe, wie Methylenchlorid, Chloroform und Tetrachlorkohlenstoff.

Die Temperaturen können bei dem obigen Verfahren zur Herstellung von Cyclopropylketonen der Formel (VIII) innerhalb eines bestimmten Bereiches variiert werden. Im allgemeinen arbeitet man bei Temperaturen zwischen -10 °C und +60 °C, vorzugsweise zwischen 0 °C und +40 °C.

Bei der Durchführung des obigen Verfahrens zur Herstellung von Cylcopropylketonen der Formel (VIII) arbeitet man im allgemeinen unter Normaldruck. Es ist jedoch auch möglich, unter erhöhtem oder vermindertem Druck zu arbeiten.

Bei der Durchführung des obigen Verfahrens zur Herstellung von Cyclopropylketonen der Formel (VIII) setzt man auf 1 Mol an Keton der Formel (IX) im allgemeinen eine stöchiometrische Menge oder auch einen geringen Überschuß an Chlorierungs- oder Bromierungsmittel ein. Die Aufarbeitung erfolgt nach üblichen Methoden. Im allgemeinen geht man so vor, daß man das Reaktionsgemisch nacheinander mit verdünnter, wäßriger Natriumhydrogencarbonat-Lösung und mit Wasser wäscht, dann trocknet und einengt.

Die bei dem obigen Verfahren zur Herstellung von Propanol-Derivaten der Formel (II) als Reaktionskomponenten benötigten metallorganischen Verbindungen der Formel (VI) sind bekannt oder lassen sich nach prinzipiell bekannten Methoden synthetisieren. So erhält man Verbindungen der Formel (VI), indem man Benzylhalogenide der Formel

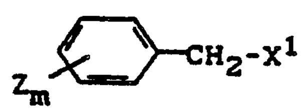
(X)

in welcher
$X^1$, Z und m die oben angegebene Bedeutung haben,
mit Magnesium in Gegenwart eines inerten Verdünnungsmittels, wie z.B. Diethylether, bei Temperaturen zwischen 0 °C und 50 °C umsetzt.

Die Benzylhalogenide der Formel (X) sind allgemein bekannte Verbindungen der organischen Chemie.

Als Verdünnungsmittel kommen bei dem obigen Verfahren zur Herstellung von Propanol-Derivaten der Formel (II) alle für derartige Umsetzungen üblichen inerten organischen Solventien in Betracht. Vorzugsweise verwendbar sind Ether, wie Diethylether, Tetrahydrofuran und Dioxan.

Die Reaktionstemperaturen können bei der Durchführung des obigen Verfahrens zur Herstellung von Propanol-Derivaten der Formel (II) innerhalb eines bestimmten Bereiches variiert werden. Im allgemeinen arbeitet man bei Temperaturen zwischen -80 °C und +50 °C, vorzugsweise zwischen -80 °C und 40 °C.

Bei der Durchführung des obigen Verfahrens zur Herstellung von Propanol-Derivaten der Formel (II) arbeitet man im allgemeinen unter Normaldruck. Es ist jedoch auch möglich, unter erhöhtem oder vermindertem Druck zu arbeiten.

Bei der Durchführung des obigen Verfahrens zur Herstellung von Propanol-Derivaten der Formel (II) setzt man auf 1 Mol an Cyclopropylketon der Formel (VIII) im allgemeinen 1 bis 1,2 Mol an metallorganischer Verbindung der Formel (VI) ein, die zweckmäßigerweise unmittelbar zuvor hergestellt und in situ weiterverarbeitet wird. Die Aufarbeitung erfolgt nach üblichen Methoden. Im allgemeinen geht man so vor, daß man zunächst ansäuert und mit Wasser versetzt, dann die organische Phase abtrennt, wäscht und nach dem Trocknen einengt.

Die bei der Durchführung des erfindungsgemäßen Verfahrens (a) als Reaktionskomponenten benötigten Azole der Formel (III) sind allgemein bekannte Verbindungen der organischen Chemie.

Als Säurebindemittel kommen bei der Durchführung des erfindungsgemäßen Verfahrens (a) alle

üblichen anorganischen und organischen Basen infrage. Vorzugsweise verwendbar sind Alkalicarbonate, wie Natrium- und Kaliumcarbonat, ferner Alkalimetallhydroxide, wie Natrium- und Kaliumhydroxid, außerdem Alkalimetallalkoholate, wie Natrium- und Kaliummethylat und -ethylat sowie Kalium-tert.-butylat, und weiterhin niedere tertiäre Alkylamine, Cycloalkylamine und Aralkylamine, wie insbesondere Triethylamin.

Als Verdünnungsmittel kommen bei der Durchführung des erfindungsgemäßen Verfahrens (a) alle üblichen inerten organischen Solventien in Betracht. Vorzugsweise verwendbar sind Nitrile, wie Acetonitril, ferner aromatische Kohlenwasserstoffe, wie Benzol, Toluol und Dichlorbenzol, außerdem Formamide, wie Dimethylformamid, sowie stark polare lösungsmittel, wie Dimethylsulfoxid und Hexamethylphosphorsäuretriamid.

Die Reaktionstemperaturen können bei der Durchführung des erfindungsgemäßen Verfahrens (a) innerhalb eines größeren Bereiches variiert werden. Im allgemeinen arbeitet man bei Temperaturen zwischen 0° C und 200° C, vorzugsweise zwischen 60° C und 150° C.

Das erfindungsgemäße Verfahren (a) wird ebenso wie die erfindungsgemäßen Verfahren (b), (c) und (d) im allgemeinen unter Normaldruck durchgeführt. Es ist jedoch jeweils auch möglich, unter erhöhtem oder vermindertem Druck zu arbeiten.

Bei der Durchführung des erfindungsgemäßen Verfahrens (a) setzt man auf 1 Mol an Propanol-Derivat der Formel (II) im allgemeinen 1 bis 4 Mol Azol der Formel (III) sowie 1 bis 3 Mol an Säurebindemittel ein. In manchen Fällen ist es zweckmäßig, unter Schutzgasatmosphäre zu arbeiten. Die Aufarbeitung erfolgt nach üblichen Methoden. Im allgemeinen geht man so vor, daß man das Reaktionsgemisch durch Abziehen des Verdünnungsmittels einengt, den verbleibenden Rückstand in einem mit Wasser wenig mischbaren organischen Lösungsmittel aufnimmt, die organische Phase wäscht und nach dem Trocknen einengt. Das verbleibende Produkt kann gegebenenfalls weiteren Reinigungsverfahren unterzogen werden.

Die bei dem erfindungsgemäßen Verfahren (b) als Ausgangsstoffe benötigten Oxirane sind durch die Formel (IV) allgemein definiert. In dieser Formel haben R, Z und m vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden.

Die Oxirane der Formel (IV) sind bisher noch nicht bekannt. Sie lassen sich herstellen, indem man Propanol-Derivat der Formel

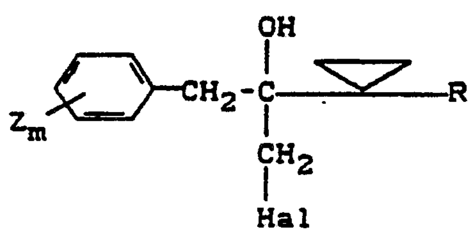

(II)

in welcher
R, Z, Hal und m die oben angegebene Bedeutung haben,
mit Basen in Gegenwart eines Verdünnungsmittels umsetzt.

Als Basen kommen bei der Herstellung von Oxiranen der Formel (IV) nach dem obigen Verfahren alle üblicherweise für derartige Umsetzungen geeigneten anorganischen und organischen Basen in Betracht. Vorzugsweise verwendbar sind alle diejenigen Basen, die bereits im Zusammenhang mit der Beschreibung des erfindungsgemäßen Verfahrens (a) als bevorzugte Säurebindemittel genannt wurden.

Die Reaktionstemperaturen können bei der Herstellung von Oxiranen nach dem obigen Verfahren innerhalb eines bestimmten Bereiches variiert werden. Im allgemeinen arbeitet man bei Temperaturen zwischen 0° C und 100° C, vorzugsweise zwischen 20° C und 60° C.

Bei der Durchführung des obigen Verfahrens zur Herstellung von Oxiranen der Formel (IV) arbeitet man im allgemeinen unter Normaldruck. Es ist jedoch auch möglich, unter erhöhtem oder vermindertem Druck zu arbeiten.

Bei der Durchführung des obigen Verfahrens zur Herstellung von Oxiranen der Formel (IV) setzt man im allgemeinen auf 1 Mol an Propanol-Derivat der Formel (II) 1 bis 3 Mol an Base ein. Die Aufarbeitung erfolgt nach üblichen Methoden.

Als Säurebindemittel und als Verdünnungsmittel kommen bei der Durchführung des erfindungsgemäßen Verfahrens (b) alle üblicherweise für derartige Umsetzungen einsetzbaren Säurebindemittel und Verdünnungsmittel infrage. Vorzugsweise verwendbar sind alle diejenigen Säurebindemittel und Verdünnungsmittel, die bereits im Zusammenhang mit der Beschreibung des erfindungsgemäßen Verfahrens (a)

als bevorzugte Säurebindemittel und Verdünnungsmittel gennant wurden.

Die Reaktionstemperaturen können bei der Durchführung des erfindungsgemäßen Verfahrens (b) innerhalb eines größeren Bereiches variiert werden. Im allgemeinen arbeitet man bei Temperaturen zwischen 0 °C und 200 °C, vorzugsweise zwischen 60 °C und 150 °C.

Bei der Durchführung des erfindungsgemäßen Verfahrens (b) setzt man auf 1 Mol an Oxiran der Formel (IV) im allgemeinen 1 bis 2 Mol an Azol der Formel (III) und 1 bis 2 Mol an Säurebindemittel ein. Die Aufarbeitung erfolgt nach üblichen Methoden.

Die bei dem erfindungsgemäßen Verfahren (c) als Ausgangsstoffe benötigten Azolylmethylketone sind durch die Formel (V) allgemein definiert. In dieser Formel haben R und X vorzugsweise diejenigen Bedeutungen, die bereits im Zusammenhang mit der Beschreibung der erfindungsgemäßen Stoffe der Formel (I) vorzugsweise für diese Reste genannt wurden.

Die Azolylmethyl-ketone der Formel (V) sind bisher noch nicht bekannt. Sie lassen sich herstellen, indem man Cyclopropylketone der Formel

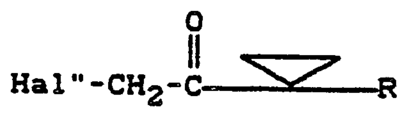

(VIII)

in welcher
R und Hal″ die oben angegebene Bedeutung haben,
mit Azolen der Formel

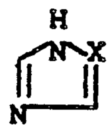

(III)

in welcher
X die oben angegebene Bedeutung hat,
in Gegenwart eines Säurebindemittels und gegebenenfalls in Gegenwart eines Verdünnungsmittels umsetzt.

Als Säurebindemittel und als Verdünnungsmittel kommen bei der Herstellungvon Azolylmethyl-ketonen der Formel (V) nach dem obigen Verfahren alle üblicherweise für derartige Umsetzungen verwendbaren Säurebindemittel und Verdünnungsmittelin Frage. Vorzugsweise verwendbar sind alle diejenigen Säurebindemittel, die bereits im Zusammenhang mit der Beschreibung des erfindungsgemäßen Verfahrens (a) als bevorzugte Säurebindemittel genannt wurden. Als Verdünnungsmittel kommen vorzugsweise Ketone, wie Aceton, und Nitrile, wie Acetonitril, in Betracht.

Die Reaktionstemperaturen können bei der Herstellung von Azolylmethyl-ketonen der Formel (V) nach dem obigen Verfahren innerhalb eines größeren Bereiches variiert werden. Im allgemeinen arbeitet man bei Temperaturen zwischen 0 °C und 120 °C, vorzugsweise zwischen 20 °C und 100 °C.

Das obige Verfahren zur Herstellung von Azolylmethylketonen der Formel (V) wird im allgemeinen unter Normaldruck durchgeführt.

Bei der Durchführung des obigen Verfahrens zur Herstellung von Azolylmethyl-ketonen der Formel (V) setzt man auf 1 Mol an Cyclopropylketon der Formel (VIII) im allgemeinen auf 1 bis 4 Mol an Azol der Formel (III) sowie 1 bis 3 Mol an Säurebindemittel ein. Die Aufarbeitung erfolgt nach üblichen Methoden.

Die bei dem erfindungsgemäßen Verfahren (c) als Reaktionskomponenten benötigten metallorganischen Verbindungen der Formel (VI) wurden bereits im Zusammenhang mit der Beschreibung des Verfahrens zur Herstellung der Propanol-Derivate der Formel (II) erwähnt.

Bei der Durchführung des erfindungsgemäßen Verfahrens (c) kommen als Verdünnungsmittel alle üblichen inerten organischen Solventien in Betracht. Vorzugsweise verwendbar sind Ether, wie Diethylether, Tetrahydrofuran und Dioxan.

Die Reaktionstemperaturen können auch bei der Durchführung des erfindungsgemäßen Verfahrens (c) in einem größeren Bereich variiert werden. Im allgemeinen arbeitet man bei Temperaturen zwischen -80 °C und +60 °C, vorzugsweise zwischen -70 °C und +50 °C.

Bei der Durchführung des erfindungsgemäßen Verfahrens (c) setzt man auf 1 Mol an Azolylmethylketon der Formel (V) im allgemeinen 0,8 bis 1 Mol an metallorganischer Verbindung der Formel (VI), die zweckmäßigerweise unmittelbar zuvor hergestellt und in situ weiterverarbeitet wird. Die Aufarbeitung erfolgt

nach üblichen Methoden.

Die bei dem erfindungsgemäßen Verfahren (d) als Ausgangsstoffe benötigten Azolylmethylcyclopropyl-Derivate der Formel (Ia) sind erfindungsgemäße Verbindungen. Ihre Überführung in die entsprechenden Alkoholate erfolgt in allgemein bekannter Weise, indem man mit geeigneten starken Basen, wie Alkalimetallamiden oder -hydriden, quarternären Ammonium-hydroxiden oder Phosphonium-hydroxiden in einem inerten Verdünnungsmittel, wie zum Beispiel Dioxan, bei Raumtemperatur umsetzt. Demgemäß steht $R^3$ in den Verbindungen der Formel (Ib) vorzugsweise für ein Alkalimetallkation, wie ein Natrium- oder Kaliumkation, oder für ein quarternäres Ammonium- oder Phosphoniumkation.

Die bei dem erfindungsgemäßen Verfahren (d) außerdem als Ausgangsstoffe benötigten Halogenverbindungen sind durch die Formel (VII) allgemein definiert. In dieser Formel steht $R^4$ vorzugsweise für die Bedeutungen, die bereits im Zusammenhang mit der Beschreibung der erfindungsgemäßen Stoffe der Formel (I) für den Substituenten $R^1$ genannt wurden, mit Ausnahme der Bedeutung von Wasserstoff. Hal steht vorzugsweise für Chlor, Brom oder Iod.

Die Halogenverbindungen der Formel (VII) sind bekannt oder lassen sich nach im Prinzip bekannten Methoden herstellen.

Als Verdünnungsmittel kommen bei der Durchführung des erfindungsgemäßen Verfahrens (c) inerte organische Lösungsmittel in Frage. Hierzu gehören vorzugsweise Ether, wie Diethylether oder Dioxan; aromatische Kohlenwasserstoffe, wie Benzol; in einzelnen Fällen auch chlorierte Kohlenwasserstoffe, wie Chloroform, Methylenchlorid oder Tetrachlorkohlenstoff; sowie Hexamethylphosphorsäuretriamid.

Die Reaktionstemperaturen können bei der Durchführung des erfindungsgemäßen Verfahrens (d) in einem größeren Bereich variiert werden. Im allgemeinen arbeitet man zwischen 0° C und 120° C, vorzugsweise zwischen 20° C und 100° C.

Bei der Durchführung des erfindungsgemäßen Verfahrens (d) setzt man zunächst Hydroxy-Verbindungen der Formel (Ia) mit starken Basen zu den entsprechenden Alkoholaten der Formel (Ib) um. In der danach folgenden Stufe setzt man auf 1 Mol eines Alkoholates der Formel (VII) vorzugsweise 1 bis 2 Mol Halogenverbindung der Formel (V) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform wird zweckmäßigerweise so verfahren, daß man von einer Hydroxy-Verbindung der Formel (Ia) ausgeht, letztere in einem geeigneten organischen Lösungsmittel mittels Alkalimetall-hydrid oder Alkalimetall-amid in das Alkalimetallalkoholat überführt und letzteres ohne Isolierung sofort mit einer Halogenverbindung der Formel (VII) umsetzt, wobei unter Austritt von Alkalimetallhalogenid die erfindungsgemäßen Verbindungen der Formel (I) in einem Arbeitsgang erhalten werden.

Nach einer weiteren bevorzugten Ausführungsform werden zweckmäßigerweise die Herstellung der Alkoholate sowie die Umsetzung mit einer Halogenverbindung der Formel (VII) in einem Zweiphasensystem, wie beispielsweise wäßrige Natron- oder Kalilauge/Toluol oder Methylenchlorid, unter Zusatz von 0,01-1 Mol eines Phasen-Transfer-Katalysators, wie beispielsweise Ammonium- oder Phosphoniumverbindungen, durchgeführt, wobei in der organischen Phase oder an der Grenzfläche die Alkoholate mit den in der organischen Phase befindlichen Halogeniden umgesetzt werden.

Die nach den erfindungsgemäßen Verfahren erhältlichen Azolylmethyl-cyclopropyl-Derivate der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren gennant wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäße Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:

Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure re, (b) Bindemittel, z.B. Carboxymethylcellulose, Alginate, Gelatine, Polyvinylpyrrolidon, (c) Feuchthaltemittel, z.B. Glycerin, (d) Sprengmittel z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulaten können mit den üblichen gegebenenfalls Opakisierungsmittel enthaltenen Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Träger stoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs-und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungs gemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise 5 bis 150 mg/kg Körpergewicht je 24 Stunden und bei parenteraler Applikation in Gesamtmengen von etwa 2,5 bis etwa 50, vorzugsweise 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

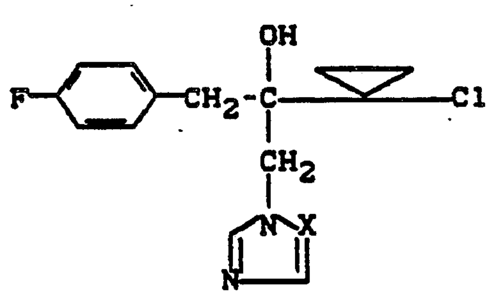

(I-1)

Unter Stickstoffatmosphäre werden 65 g (0,94 Mol) 1,2,4-Triazol und 71 g (0,63 Mol) Kalium-tert.-butylat in 160 ml absolutem Dimethylformamid vorgelegt und auf 80 °C erwärmt. Bei dieser Temperatur tropft man unter Rühren eine Lösung von 70,6 g (0,26 Mol) 1-Chlor-2-(1-chlorcyclopropyl)-3-(4-fluorphenyl)-propan-2-ol in 90 ml absolutem Dimethylformamid hinzu. Es wird 6 Stunden bei 100 °C nachgerührt und dann durch Abziehen des Verdünnungsmittels unter vermindertem Druck eingeengt. Man nimmt den Rückstand in Essigester auf, wäscht mit Wasser und zieht nach dem Trocknen über Natriumsulfat das Lösungsmittel unter vermindertem Druck ab. Das verbleibende Produkt wird über eine Kieselgel-Säule mit Chloroform als Laufmittel chromatographiert. Man erhält auf diese Weise 28,2 g (39 % der Theorie) an 1-(4-Fluorphenyl)-2-(1-chlor-cyclopropyl)-3-(1,2,4-triazol-1-yl)-propan-2-ol in Form einer Festsubstanz vom Schmelzpunkt

111 °C.

Herstellung von Vorprodukten:

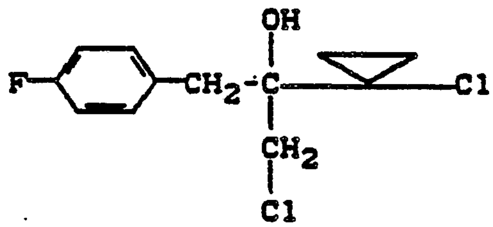

(II-1)

Eine Lösung von 65,7 g (0,35 Mol) 4-Fluorbenzyl-bromid in 430 ml absolutem Diethylether wird bei Raumtemperatur in ein Gemisch aus 9,3 g (0,38 Mol) Magnesium-Spänen in 185 ml Diethylether getropft. Man erhitzt 30 Minuten unter Rückfluß und tropft die entstandene Lösung dann bei -78 °C unter Rühren in 47,8 g (0,32 Mol) 1-Chlor-1-chloracetyl-cyclopropan ein. Das Reaktionsgemisch wird 4 Stunden bei -78 °C gerührt. Danach läßt man langsam auf 0 °C erwärmen und tropft dann eine Lösung von 31 ml Essigsäure in 300 ml Diethylether hinzu. Das entstandene Reaktionsgemisch wird auf 1.200 ml Wasser gegossen. Die organische Phase wird abgetrennt, mit wäßriger Natriumhydrogensulfit-Lösung und mit Wasser gewaschen, über Natriumsulfat getrocknet und unter vermindertem Druck eingeengt. Man erhält 70,6 g (86 % der Theorie) an 1-Chlor-2-(1-chlor-cyclopropyl)-3-(4-fluor-phenyl)-propan-2-ol in Form eines öligen Produktes.
$^1$H-NMR (200 MHz, $CDCl_3$): $\delta$ = 0,6-1,2 (m, 4H);
3,05 (d, 1H); 3,15 (d 1H); 3,75 (d, 1H); 4,03 (d, 1H);
7,0 (t, 2H); 7,22-7,37 (m, 2H).

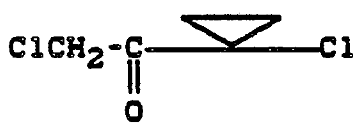

(VIII 1)

Bei Raumtemperatur werden 40,5 ml (0.5 Mol) Sulfurylchlorid unter Rühren langsam in eine Lösung von 54 g (0.46 Mol) 1-Acetyl-1-chlor-cyclopropan in 250 ml Methylenchlorid eingetropft. Es wird zunächst 14 Stunden bei Raumtemperatur und dann 30 Minuten bei 30 °C gerührt. Das Reaktionsgemisch wird dann nacheinander mit gesättigter, wäßriger Natriumhydrogencarbonat-Lösung und mit Wasser gewaschen. Danach wird die organische Phase über Natriumsulfat getrocknet und unter vermindertem Druck eingeengt. Man erhält auf diese Weise 51,5 g (74 % der Theorie) an 1-Chlor-1-chloracetyl-cyclopropan in Form einer öligen Substanz.
$^1$H-NMR (60 MHz, $CDCl_3$): $\delta$ = 1,2-1,9 (m, 4H); 4,8 (s, 2H)

Die Verbindung der Formel

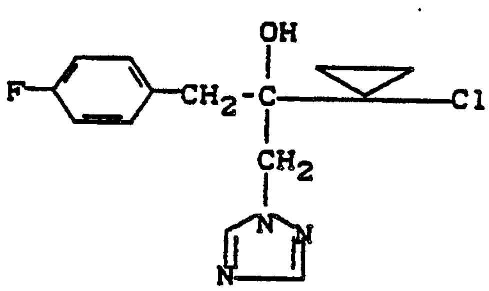

(I-1)

läßt sich auch nach dem erfindungsgemäßen Verfahren (c) herstellen, indem man (1,2,4-Triazol-1-yl-methyl)-(1-chlor-cycloprop-1-yl)-keton mit 4-Fluor-benzyl-magnesium-bromid in Gegenwart von Diethylether umsetzt.

Tabelle

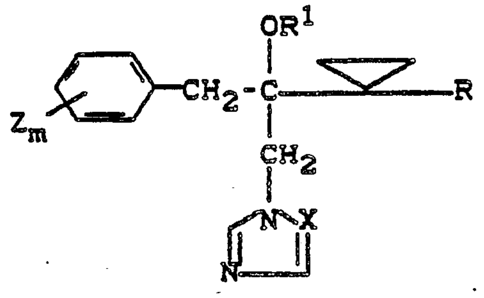

(I)

| Beispiel Nr. | $Z_m$ | $R^1$ | X | R | Schmelzpunkt (°C) |
|---|---|---|---|---|---|
| 2 | 2-F | H | N | Cl | 103 |
| 3 | 2,4-$F_2$ | H | N | Cl | 117 |
| 4 | 2-Cl | H | N | Cl | 108 |
| 5 | 3,4-$Cl_2$ | H | N | Cl | 80-92 |
| 6 | - | H | N | Cl | 81 |
| 7 | 4-Cl | H | N | Cl | 97 |
| 8 | 2,4-$Cl_2$ | H | N | Cl | 159 |
| 9 | 4-$CH_3$ | H | N | Cl | 132 |
| 10 | 3,4-$F_2$ | H | N | Cl | 98 |
| 11 | 4-F | H | CH | Cl | 171 |

In den folgenden Verwendungsbeispielen wurden die Verbindungen der nachstehend angegebenen Formeln als Vergleichssubstanzen eingesetzt:

Verwendungsbeispiele

In dem folgenden in-vivo-Test werden die nachstehend angegebenen Verbindungen als Vergleichssubstanzen eingesetzt:

Herstellung des Ausgangsstoffes der Formel:

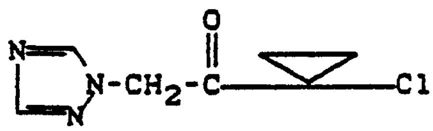

(V-1)

In eine Lösung von 50 g Kaliumcarbonat und 35 g 1,2,4-Triazol in 200 ml Aceton wird bei Raumtemperatur unter Stickstoffatmosphäre und unter Rühren eine Lösung von 60 g 1-Acetyl-1-chlor-cyclopropan in 50 ml Aceton eingetropft. Man erhitzt 8 Stunden unter Rückfluß, engt dann durch Abziehen des Verdünnungsmittels unter vermindertem Druck ein, nimmt dem Rückstand in einem Gemisch aus Essigester und Toluol auf, wäscht mit Wasser, trocknet über Natriumsulfat und engt unter vermindertem Druck ein. Man erhält auf diese Weise 38,9 g an (1,2,4-Triazol-1-yl-methyl)-(1-chlorcycloprop-1-yl)-keton in Form einer Festsubstanz vom Schmelzpunkt 79° C.

$^1$H-NMR (200 MHz, $CDCl_3$):

δ = 1,50 (m, 2H), 1,78 (m, 2H),

5,62 (s, 2H), 7,98 (s, 1H),

8,14 (s, 1H).

Nach den im Beispiel 1 und in der Beschreibung angegebenen Methoden werden auch die in der folgenden Tabelle aufgeführten Azolylmethyl-cyclopropyl-Derivate der Formel (I) hergestellt.

(A) 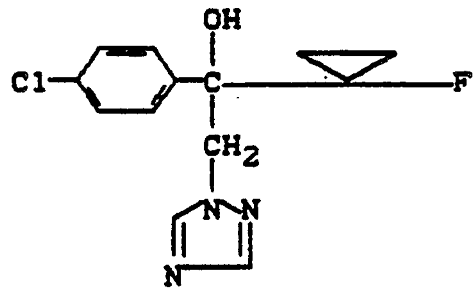

(B) 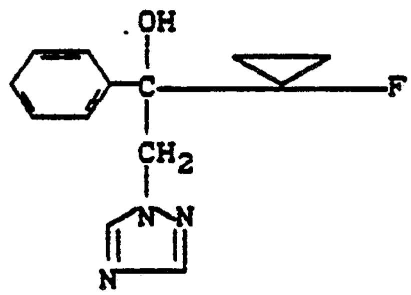

(bekannt aus EP-OS 0 180 850)

Beispiel A

Antimykotische in-vivo-Wirksamkeit (oral) bei Mäusecandidose

Versuchsbeschreibung :

Mäuse vom Typ SPF-$CF_1$ wurden intravenös mit 1 - 2 x $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 10 - 100 mg/kg Körpergewicht der Präparate oral behandelt.

Ergebnis:

Unbehandelte Tiere starben 3 bis 6 Tage post infektionem. Die Überlebensrate am 6.Tag post infektionem betrug bei unbehandelten Kontrolltieren etwa 5 %.

In diesem Test zeigen z.B. die erfindungsgemäße Verbindung (I-1) eine bessere Wirkung als die aus dem Stand der Technik bekannten Verbindungen (A) und (B).

Tabelle A

| Antimykotische in-vivo-Wirkung (oral) bei Mäuse-Candidose | |
|---|---|
| Wirkstoff | Wirkung |
| (A)(bekannt)<br>(B)(bekannt) | k.W.<br>k.W. |
| Verbindungen gemäß Herstellungsbeispiel | |
| (I-1) | + + + + + |
| Zeichenerklärung: | |
| + + + + + = sehr gute Wirkung = 90 % Überlebende am 6.Tag.p.i.<br>+ + + + = gute Wirkung = 80 % Überlebende am 6.Tag.p.i.<br>+ + + = Wirkung = 60 % Überlebende am 6.Tag.p.i.<br>+ + = schwache Wirkung = 40 % Überlebende am 6.Tag.p.i.<br>+ = Spur Wirkung = unter 40 % Überlebende am 6. Tag p.i.<br>k.W. = kein Unterschied zur unbehandelten Infektionskontrolle | |

Beispiel B / Formulierungen

| 1.) Lösung: | |
|---|---|
| Wirkstoff gemäß Formel (I):<br>Alkohol, rein (96 %ig) :<br>Isopropylmyristat : | 10 g<br>300 g<br>526 g<br>836 g |

| 2.) Creme: | |
|---|---|
| Wirkstoff gemäß Formel (I): | 10 g |
| Aralcel 60 : | 20 g |
| (Sorbitan-monostearat) | |
| Tween 60 : | 15 g |
| (Polyoxyethylen (2) -sorbitan-monostearat) | |
| Walrat, künstlich : | 30 g |
| (Mischung vom Estern von gesättigten Fettsäuren $C_{14}$-$C_{18}$ und Fettalkoholen $C_{14}$-$C_{18}$, | |
| Lanette O : | 100 g |
| Eutanol G : | 135 g |
| (2-Octyl-dodecanol) | |
| Benzylalkohol : | 10 g |
| Wasser, entmineralisiert : | 680 g |
| | 1000 g |

## Ansprüche

1. Azolylmethylcyclopropyl-Derivate der allgemeinen Formel

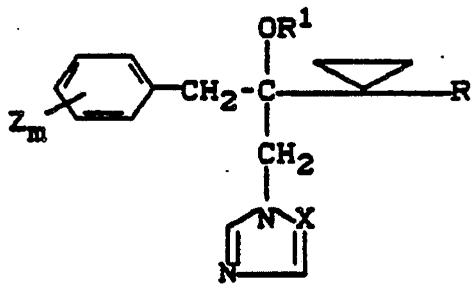

in welcher
R für Halogen, Alkyl, gegebenenfalls substituiertes Phenyl oder für die Gruppierung -Y-$R^2$ steht, worin
Y für Sauerstoff, Schwefel, SO oder $SO_2$ steht und
$R^2$ für gegebenenfalls substituiertes Phenyl steht,
$R^1$ für Wasserstoff, Alkyl oder Acyl steht,
X für Stickstoff oder eine CH-Gruppe steht,
Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und
m für die zahlen 0, 1, 2 oder 3 steht,
sowie deren Säureadditions-Salze zur Bekämpfung von Krankheiten.

2. Azolylmethylcyclopropyl-Derivate nach Anspruch 1,
in denen
R für Fluor, Chlor, Brom, Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung -Y-$R^2$, worin
Y für Sauerstoff, Schwefel, SO oder $SO_2$ steht und
$R^2$ für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht,

$R^1$ für Wasserstoff, Alkyl mit 1 bis 6 Kohlenstoffatomen oder Alkylcarbonyl mit 1 bis 6 Kohlenstoffatomen in der Alkylgruppe,
X für Stickstoff oder eine CH-Gruppe,
Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy und
m für die Zahlen 0, 1, 2 oder 3,
sowie deren Säureadditions-Salze zur Bekämpfung von Krankheiten.

3. Azolylmethylcyclopropyl-Derivate nach Anspruch 1, in denen
R für Fluor, Chor, Brom, Methyl, Ethyl, gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder für die Gruppierung -Y-$R^2$ steht, worin
Y für Sauerstoff, Schwefel, SO oder $SO_2$ steht und
$R^2$ für gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht,
$R^1$ für Wasserstoff, Methyl, Ethyl, n-Propyl, Isopropyl, n-Butyl, Isobutyl, Methylcarbonyl, Ethylcarbonyl, n-Propylcarbonyl, Isopropylcarbonyl, n-Butylcarbonyl und Isobutylcarbonyl steht,
X für Stickstoff oder eine CH-Gruppe steht,
Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy steht und
m für die Zahlen 0, 1, 2 oder 3 steht,
sowie deren Säureadditions-Salze zur Bekämpfung von Krankheiten.

4. 1-(4-Fluorphenyl)-2-(1-chlor-cyclopropyl)-3-(1,2,4-triazol-1-yl)-propan-2-ol

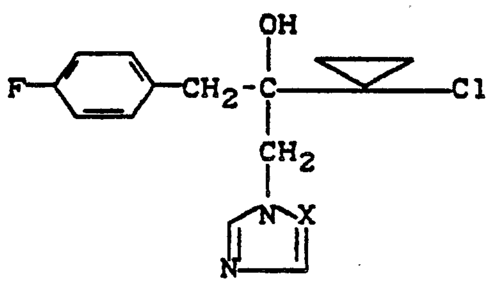

sowie dessen Säureadditionssalze zur Bekämpfung von Krankheiten.

5. Azolylmethylcyclopropyl-Derivate nach Ansprüchen 1-4 zur Bekämpfung von Mykosen.

6. Arzneimittel enthaltend Azolylmethylcyclopropyl-Derivate nach Ansprüchen 1-4.

7. Antimykotische Mittel enthaltend Azolylmethylcyclopropyl-Derivate nach Ansprüchen 1-4.

8. Verwendung der Azolylmethylcyclopropyl-Derivate nach Ansprüchen 1-4 bei der Herstellung von Arzneimitteln zur Bekämpfung von Krankheiten.

9. Verwendung der Azolylmethylcyclopropyl-Derivate nach Ansprüchen 1-4 bei der Herstellung von Arzneimitteln zur Bekämpfung von Mykosen.